# EUROPÄISCHE PATENTSCHRIFT

(11) **EP 1 562 885 B1**
(45) Veröffentlichungstag und Bekanntmachung des Hinweises auf die Patenterteilung: **05.09.2007**
(21) Anmeldenummer: 03778290.1
(22) Anmeldetag: 28.10.2003
(51) Int. Cl.: C07C 45/54, C07C 49/203, C07C 403/16

(54) **KONTINUIERLICHES VERFAHREN ZUR HERSTELLUNG VON PSEUDOJONONEN UND JONONEN**
CONTINUOUS PROCESS FOR PRODUCING PSEUDOIONONES AND IONONES
PROCEDE POUR PRODUIRE EN CONTINU DES IONONES ET PSEUDOIONONES

(30) Priorität: 07.11.2002 DE 10252259
(43) Veröffentlichungstag der Anmeldung: 17.08.2005
(73) Patentinhaber: BASF Aktiengesellschaft, 67056 Ludwigshafen (DE)
(72) Erfinder: DOBLER, Walter, 68723 Schwetzingen (DE); BAHR, Nicolaus, 69126 Heidelberg (DE); BREUER, Klaus, 67122 Altrip (DE); KINDLER, Alois, 67269 Grünstadt (DE)
(86) Internationale Anmeldenummer: PCT/EP2003/011926
(87) Internationale Veröffentlichungsnummer: WO 2004/041764

(56) Entgegenhaltungen:
- EP-A- 0 062 291
- EP-A- 0 295 361
- WO-A-97/43254
- US-A- 3 480 677
- DATABASE WPI Section Ch, Week 198032 Derwent Publications Ltd., London, GB; Class B05, AN 1980-56547C XP002267975 & SU 704 938 A (KALUGA SYNTH PERFUME), 28. Dezember 1979 (1979-12-28)

## Beschreibung

Die vorliegende Erfindung betrifft ein kontinuierliches Verfahren zur Herstellung von Psoudojononen und deren gegebenenfalls anschließende Zyklisierung zu Jononen. Bevorzugt wird das Verfahren zur Herstellung von Verbindungen wie 6-Methylhepta-3,5-dien-2-on, Pseudojonon, Methylpseudojonon, Dimethylpseudojonon, Pseudoiron, Methylpseudoiron und Dimethylpseudoiron sowie deren Zyklisierungsprodukten α-, β- und γ-Jonon, α-, β- und γ-Methyljonon (N-Form, iso-Form bzw. Gemische) und Homologen. Diese Stoffe sind von großer wirtschaftlicher Bedeutung als Riechstoffe und Riechstoffzwischenprodukte. Pseudojonon selbst ist darüber hinaus bedeutendes Zwischenprodukt für die Herstellung der Vitamine E und A sowie von Carotinoiden. β-Jonon ist ein bedeutendes Zwischenprodukt für die Herstellung von Vitamin A und Carotinoiden.

Zur Herstellung von Pseudojononen aus Citral sind zahlreiche Methoden bekannt.

In SU 704938 wird ein Verfahren zur Herstellung von Pseudojonen durch Reaktion von Citral mit einem 12- bis 20-fachen Überschuss von Aceton in Gegenwart wässriger Natronlauge beschrieben. Das molare Verhältnis Natronlauge: Litral beträgt 0.15-0.45:1. Das Volumenverhältnis Aceton: Wasser beträgt 1:0.15-0.45.

In PL 147748 wird ein Verfahren zur Herstellung von Jononen durch Kondensation von Citral und Aceton an basischen Ionentauschern bei 66 °C beschrieben. Hiernach werden Aceton und Citral für 5 Stunden diskontinuierlich in einem Kolben mit dem Katalysator gerührt. Nachteilig an diesem Verfahren sind die sehr geringen Raum-Zeit-Ausbeuten.

In DE-A 33 19430 wird die Herstellung von höheren Ketonen durch Kondensation von Methylketonen und ungesättigten Aldehyden an gemischten Metallkatalysatoren in Gegenwart von Wasserstoff bei 100 bis 280 °C und 10 bis 60 bar in einem Rohrreaktor gelehrt.

Ein Verfahren zur Herstellung von Pseudojononen durch Umsetzung von Citral mit Aceton unter Verwendung von LiOH als Katalysator wird in US 4,874,900 beschrieben. Hiernach wird die Reaktion batch-weise oder kontinuierlich bei Temperaturen von -20 bis 240°C durchgeführt. Der Druck wird so eingestellt, dass das Reaktionsgemisch bei der entsprechenden Temperatur in der flüssigen Phase bleibt. Beim diskontinuierlichen Betrieb werden die Reaktanten in einem Kessel verrührt und der Katalysator nach Beendigung der Reaktion abfiltriert, während bei der kontinuierlichen Fahrweise die vorgemischten Reaktanten durch eine mit Katalysator gefüllte Kolonne gepumpt werden. In beiden Fällen wird das Reaktionsgemisch nach Reaktionsende mit CO₂ neutralisiert und das überschüssige Keton abdestilliert. Bei diesem Verfahren werden bei einem Molverhältnis Aceton zu Citral von 20 Ausbeuten von 89,5% Citral erzielt. Diese geringen Ausbeuten sind für ein großtechnisches Verfahren unbefriedigend.

In DE-A 31 14071 wird ein Verfahren zur Herstellung von Pseudjononen durch Umsetzung eines Aldehyds mit einem Überschuss eines Ketons bei erhöhter Temperatur beschrieben.

Auch für die anschließende Zyklisierung von Pseudojononen zu Jononen sind im Stand der Technik zahlreiche Methoden bekannt. So ist bekannt, dass bei der Zyklisierung von Pseudojonon mit Säuren, wie konzentrierter Schwefelsäure oder Phosphorsäure Gemische von α- und β-Jononen erhalten werden. Das Verhältnis der Mengen, in welchen diese Verbindungen entstehen, ist stark von den Bedingungen, unter denen die Reaktion stattfindet abhängig.

Bei Zyklisierungen mit konzentrierter Schwefelsäure, die stark exotherm verlaufen, ist es wichtig die entstehende Reaktionswärme möglichst schnell abzuführen, um örtliche Überhitzungen zu vermeiden. Zu diesem Zweck werden dem Reaktionsgemisch bei den bekannten Verfahren Verdünnungsmittel zugegeben.

Aufgabe der vorliegenden Erfindung war die Entwicklung eines Verfahrens zur Herstellung von Pseudojononen und einer sich gegebenenfalls daran anschließenden Zyklisierung zu den entsprechenden Jononen, das im Vergleich zum Stand der Technik weniger Einsatzstoffe braucht und pro Einsatzstoff mehr Produkt erzeugt.
1. Die Aufgabe wurde erfindungsgemäß gelöst durch die Bereitstellung eines kontinuierlichen Verfahrens zur Herstellung von Pseudojononen der allgemeinen Formeln I bzw. I' sowie ihrer isomeren 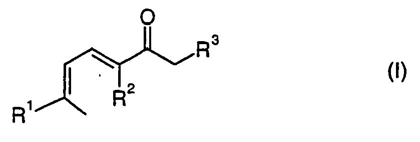 bzw. 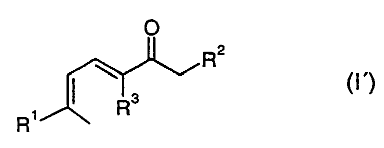 wobei R¹
   CH₃ oder 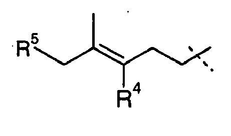 bedeutet,
   R², R³ Wasserstoff, CH₃ oder C₂H₅ bedeuten,
   R⁴, R⁵ Wasserstoff oder CH₃ bedeuten,
   durch Umsetzen eines Aldehyds der Formel (II), 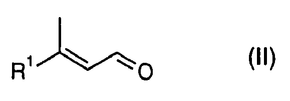 mit einem Überschuss eines Ketons der allgemeinen Formel (III) 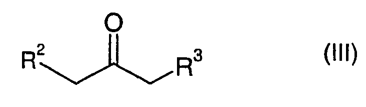
wobei R¹, R² und R³ die oben angegebenen Bedeutungen besitzen, in Gegenwart von Wasser und Alkalihydroxid bei erhöhter Temperatur in homogener Lösung, das dadurch gekennzeichnet ist, dass
a) die Durchmischung der homogenen Lösung von Aldehyd, Keton und wässriger Alkalilauge bei einer Temperatur von 10 bis 120 °C stattfindet, dann
b) das nicht im Reaktionsgemisch gelöste Wasser und Alkalihydroxid abgetrennt werden,
c) anschließend das homogene Reaktionsgemisch unter Vermeidung von Rückvermischung bei einer Temperatur, die 10 bis 120°C oberhalb des Siedepunktes der am niedrigsten siedenden Komponente und einem Dampfdruck p von 10⁶ bis 10⁷ Pa durch einen Reaktor geleitet wird, der eine Verweilzeit von 2 bis 300 Minuten ermöglicht,
d) das Reaktionsgemisch unter Entspannung abgekühlt wird,
e) das Keton mit Dampf im Gegenstrom aus dem Reaktionsgemisch entfernt wird und
f) das Rohprodukt getrocknet und über eine Rektifikationskolonne von überschüssigem Aldehyd und Nebenkomponenten befreit wird.

Bevorzugt wird das erfindungsgemäße Verfahren zur Herstellung von 6-Methylhepta-3,5-dien-2-on, Pseudojonon, Methylpseudojonon, Dimethylpseudojonon, Pseudoiron, Methylpseudoiron und Dimethylpseudoiron und ihrer Isomeren eingesetzt.

Als erfindungsgemäß eingesetzte Aldehyde werden bevorzugt Citral, Citronellal und 2,6-Dimethyloctanal, aber auch jeder geradkettiger oder verzweigter gesättigter oder auch ungesättigter Aldehyd mit 1 bis 10 C-Atomen, und als Ketone bevorzugt Aceton, 2-Butanon, oder 2 bzw. 3-Pentanon eingesetzt.

Unter wässriger Alkalilauge versteht man eine wässrige Lösung von Kaliumhydroxid, Natriumhydroxid oder Lithiumhydroxid, vorzugsweise jedoch Natriumhydroxidlösung. Die Konzentration des eingesetzten Alkalihydroxids liegt zwischen 0,005 und 50 Gew.-%, vorzugsweise zwischen 1 und 15 Gew.-%.

Unter den Isomeren versteht man sämtliche möglichen Stellungsisomere oder Doppelbindungsisomere der Pseudojonone oder Jonone.

In dem erfindungsgemässen Verfahren wird dem homogenen Gemisch der Edukte Aldehyd, Keton und Wasser bei 10 bis 120°C, bevorzugt bei Temperaturen kleiner als 50 °C nur soviel wässrige Alkalilauge zugegeben, wie sich nach inniger Durchmischung homogen löst. Eventuell sich abscheidendes Wasser und Alkalihydroxid wird abgetrennt, bevor das homogene Reaktionsgemisch unter Vermeidung von Rückvermischung bei einer Temperatur, die 10 bis 120°C oberhalb des Siedepunktes der am niedrigsten siedenden Komponente liegt und einem Druck p von 10⁶ bis 10⁷ Pa, wobei p der Dampfdruck des Reaktionsgemisches bei der Reaktionstemperatur ist, durch einen Reaktor geleitet wird, der eine Verweilzeit von 2 bis 300 Minuten, vorzugsweise 5 bis 30 Minuten ermöglicht. Das Reaktionsgemisch wird durch Entspannung abkühlt, wobei ein Teil des Ketonüberschusses verdampft und der Rückführung zugeführt werden kann, das Keton dann mit Dampf im Gegenstrom aus dem Reaktionsgemisch entfernt, wobei der Dampf soviel einer verdampfbaren Säure enthält, dass die Katalysatorbase neutralisiert wird und sich ein pH-Wert von 4 bis 9 einstellt. Anschließend wird das Rohprodukt getrocknet und über eine Rektifikationskolonne, vorzugsweise über eine Trennwandkolonne, wie sie z.B. in DE-A 3302525 oder in EP-A 804 951 offenbart ist, von überschüssigem Aldehyd und Nebenkomponenten befreit.

Ein weiterer Gegenstand der Erfindung ist ein kontinuierliches Verfahren zur Herstellung von Jononen der allgemeinen Formeln (IV), (V) und (VI) sowie ihrer Isomeren, das dadurch gekennzeichnet ist, dass die nach dem erfindungsgemäßen Verfahren erhaltene Pseudojonone zu Jononen der allgemeinen Formeln (lV)-(Vl) 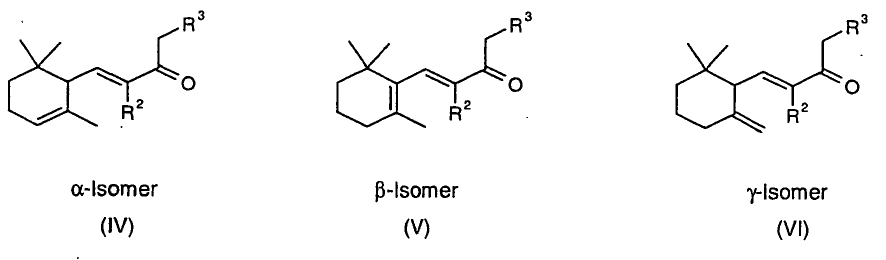 umgesetzt werden.

Es war überraschend, dass die Bildung von Neben- und Zersetzungsprodukten, die als Nebenreaktion bei der heterogenen Katalyse durch Alkalihydroxid vor allem die Aufarbeitung des Reaktionsgemisches auftritt, zurückgedrängt werden kann, wenn das Gemisch aus Keton und Aldehyd unterhalb der Prozeßtemperatur im Reaktor nur mit soviel Alkalilauge versetzt wird, wie homogen eingelöst werden kann, und das homogene, mit wässriger Alkalilauge gesättigte Gemisch in einem Rohrreaktor ohne weitere Durchmischung unter Eigendruck auf die gewünschte Reaktionstemperatur gebracht wird.

Vorteilhaft ist es, eventuell auftretende, nicht ins Gemisch eingelöste und somit überschüssige Alkalilauge am Reaktoreingang abzutrennen. Dies kann an einem Abscheider erfolgen, der entweder dem Reaktor vorgeschaltet ist oder in den Sumpf des Reaktors integriert ist. Man kann auch vorteilhaft überschüssiges Wasser aus dem rückzuführenden Keton abtrennen, indem man dem Reaktionsgemisch hochkonzentrierte, d.h. etwa 10 bis 50, vorzugsweise 35 bis 45 %ige Alkalilauge zudosiert, welche dem Reaktionsgemisch Wasser entzieht und die erforderliche Menge an Alkalihydroxid in das Reaktionsgemisch einlöst.

Die Reaktion wird mit einem 5 bis 50-fachen, vorzugsweise mit einem 20 bis 25-fachen molaren Überschuss an Keton gefahren, um eine optimale Ausbeute bezüglich des eingesetzten Aldehyds zu erzielen. Der nicht umgesetzte Anteil Keton wird nach der Reaktionsstrecke bei einem Druck von 10⁷ bis 10⁹ mPa_{abs} abgetrennt und dem Frischketon zur Synthese wieder zugeführt.

Überraschenderweise kommt auch dem Wassergehalt des Aldehyd-Keton-Gemisches eine besondere Bedeutung zu. Dieser bestimmt offensichtlich die.Menge an Alkalihydroxyd, welches sich im Aldehyd-Keton-Gemisch homogen einlösen kann. Der Wassergehalt des Aldehyd-Keton-Gemisches sollte zwischen 1 und 15 Gew.-% liegen. Die eingelöste Menge an Alkalihydroxyd bestimmt wiederum die Umsetzungsgeschwindigkeit, beeinflusst allerdings auch die Rate an unerwünschten Nebenprodukten. Dies steht im Übereinstimmung mit der Tatsache, dass die Abtrennung von überschüssiger Lauge vor dem Reaktor vorteilhaft ist. Im Gegensatz zum Stand der Technik wird dadurch erreicht, dass ins Reaktionsgemisch gegen Ende der Reaktion infolge der Zunahme des Wassergehaltes durch das Reaktionswasser kein weiteres Alkalihydroxyd eingelöst wird, welches in dieser Phase die Nebenproduktbildung begünstigt. Letztere spielt vor allem bei empfindlichen ungesättigten Aldehyden wie z.B. Citral eine bedeutende Rolle und senkt die Ausbeute.

Das Wasser wird vorteilhaft über den Anteil der Ketonkomponente in das Verfahren eingetragen, welcher durch die Wasserdampfstrippung des Reaktionsgemisches nach dem Reaktor generiert wird. Es ist von wirtschaftlicher Bedeutung, dass dadurch der Ketonüberschuss mit geringem technischen und energetischen Aufwand abgetrennt werden kann, da eine aufwändige Trocknung vor der Rückführung überflüssig wird. Alternativ kann auch mit einem wasserfreien Gemisch aus Aldehyd und Keton gefahren werden und das benötigte Wasser (etwa 1 bis 15 Gew.-%) eingemischt werden, in dem eine sehr verdünnte Alkalihydroxydlösung verwendet wird. Umgekehrt lässt sich ein Gemisch aus Aldehyd und Keton mit sehr hohem Gehalt an Wasser verwenden, wenn eine konzentrierte Alkalihydroxydlösung zugemischt wird. Dabei ist eine tiefere Mischtemperatur erforderlich, um den unkontrollierten Beginn der Reaktion zu vermeiden. Gleichzeitig steigt der Verbrauch an Alkalihydroxid, da dieses nur zum Teil in die organische Phase übertritt. Zum Teil entzieht es dem Aldehyd-Keton-Gemisch Wasser und muss abgetrennt und entsorgt werden.

Die homogene Reaktionslösung wird unter Eigendruck in einem Rohrreaktor erhitzt, wobei die Reaktionstemperatur bei gegebener Verweilzeit so eingestellt wird, dass der Umsatz der Aldehydkomponente 60 bis 98%, vorzugsweise 85 bis 95% beträgt, wobei der nicht umgesetzte Aldehyd abgetrennt und in die Umsetzung rückgeführt wird. Der Rohrreaktor ist dabei so dimensioniert, dass die mittlere Verweilzeit zwischen 2 und 300 Minuten, vorzugsweise zwischen 5 und 30 Minuten liegt, in der Art, dass es möglichst zu keiner Rückvermischung kommt. Höhere Umsätze erfordern ein überproportionales Anheben der Reaktionstemperatur, was die Nebenproduktbildung begünstigt. Geringere Umsätze ermöglichen eine niedrigere Reaktionstemperatur, welche die Nebenproduktrate zurückdrängt, erhöhen jedoch die Rückführströme Keton und Aldehyd und damit den Energiebedarf des Verfahrens.

Im Rohrreaktor muss die Rückvermischung minimiert werden. Dies kann erreicht werden durch einen ausreichend großen Reaktordurchmesser, um Turbulenzen zu vermeiden, oder aber auch durch Laminar-Flow-Einbauten jeglicher Art. Dies ist überraschend und steht im Widerspruch zum Stand der Technik, wo z.B: nach DE-A 31 14 071 Rohrreaktoren derart beschaffen sein müssen, dass unter den Reaktionsbedingungen eine ausreichend turbulente Strömung herrscht.

Das Reaktionsgemisch wird auf Normaldruck entspannt, wobei es sich über die Verdampfung eines Teils des überschüssigen Ketons abkühlt. Das restliche Keton wird in einer Gegenstromkolonne mit Wasserdampf, dem eine äquimolare Menge von einer flüchtigen Säure beigemischt ist, ausgetrieben, wobei die Katalysatorbase neutralisiert und durch das Kondensat verdünnt wird. Durch Verwendung von Kolonnenpackungen wird sicher gestellt, dass am Kolonnenkopf neben Keton und Wasser keine nennenswerten Mengen an weiteren Produkten anfallen, wobei der Rücklauf zur Kolonne vorteilhaft so eingestellt wird, dass das Keton mit der gewünschten Wassermenge abgezogen werden kann. Die Säuremenge wird vorteilhaft so bemessen, dass sich an dieser Stelle der für die weitere Aufarbeitung günstigste pH-Wert von 4 bis 9 einstellt. Nach Abtrennung der wässrigen Phase wird das Rohprodukt getrocknet, in dem es aufgeheizt und in einen Flashbehälter gedüst wird, der unter vermindertem Druck gehalten wird. Von dort wird in eine Rektifikationskolonne überführt, in der das ungesättigte Keton unter vermindertem Druck von Verunreinigungen gereinigt sowie das nicht umgesetzte Aldehyd abgetrennt und von dort der Rückführung zugeführt wird. Die Rückführung geschieht vorteilhaft in einer Trennwandkolonne wie in EP-A 804 951 beschrieben, wobei hier vorzugsweise 2 Seitenabzüge vorliegen, um beide Hauptfraktionen (Produkt und Aldehyd) in einem Schritt in ausreichender Reinheit zu erhalten.

Ganz besondere Bedeutung hat das oben beschriebene Verfahren, wenn man Citral als Aldehydkomponente und 2-Butanon als Ketonkomponente einsetzt. Es bildet sich ein charakteristisches Gemisch aus 70 - 97 % n-Methylpseudojonon und 3 - 30% iso-Methylpseudojonon, welches sich zu einem charakteristischen Gemisch aus Methyljononen zyklisieren lässt. Methyljonone gibt es in den unterschiedlichsten lsomerenverhältnissen. Jedes von ihnen wird von der Riechstoffindustrie in großer Menge zur Herstellung von industriellen Parfums eingesetzt. Da jedes lsomerenverhältnis eine etwas andere Duftnote besitzt, ist die Reproduzierbarkeit eines einmal eingesetzten Isomerenverhältnisses von höchster Bedeutung.

Zur Herstellung der korrespondierenden Jonone wird das erhaltene Gemisch aus Pseudojononen mit hochprozentiger, d.h. etwa 50 bis etwa 98 %ige Schwefelsäure in Gegenwart eines unter den Reaktionsbedingungen inerten Verdünnungsmittels umgesetzt, und zwar vorteilhafterweiser wie in DE 196 19 557 beschrieben. In Abweichung dazu sind Zyklisiertemperaturen von < 20°C und eine Schwefelsäurekonzentration von < 90% vorteilhaft, wenn zwischen Zyklisierung und Hydrolyse eine Verweilzeit von > 10 Sekunden eingehalten wird. Im Falle von Methyliononen ist eine Schwefelsäurekonzentration von etwa 89% bei einer Verweilzeit von etwa 2 min bei etwa 25°C vorteilhaft, wobei fast ausschließlich die β-Isomeren erhalten werden, während die Bildung von α-n- und γ-n-Methyliononen auf etwa 1 % zurückgedrängt wird. Im Falle von Pseudojonon wird mit 89%iger Schwefelsäure in hoher Ausbeute nahezu ausschließlich β-Jonon gebildet, während α-Jonon und γ-Jonon nur im 1%-Bereich liegen und problemlos bei der Reindestillation über Kopf abgetrennt werden können.

Die nachfolgenden Beispiele sollen die Erfindung näher erläutern, ohne sie jedoch darauf zu beschränken.

### Beispiel 1

### Herstellung von Pseudojonon

370 kg/h Citral (2,43 kmol/h), ca. 26kg/h Rückcitral, 3.800 kg/h wässriges 95%iges Aceton und 30 kg/h 5%ige wässrige NaOH werden vermischt. Es entsteht eine homogene Lösung, die vorsorglich über einen Phasenabscheider geleitet wird. Ist der Wasseranteil oder auch der NaOH-Anteil höher als angegeben, kann sich nach dem Mischprozess eine wässrige Phase abtrennen, die abgeschieden werden muss. Das Gemisch wird auf 108°C geheizt und durch einen 160 l Rohrreaktor gepumpt. Die Reaktionstemperatur erwärmt das Gemisch weiter auf ca. 112°C. Bei einer Verweilzeit von ca. 2 min wird ein Umsatz von ca. 93% erzielt.

Die Reaktionsmischung aus dem Rohrreaktor wird auf Normaldruck entspannt. Hierbei destillieren ca. 2000 l/h Aceton ab, wobei sich die Produktlösung auf ca. 60°C abkühlt. Anschließend wird im Gegenstrom mit ca. 700 kg/h Dampf von restlichem Aceton befreit. Dem Dampf werden so viel Essigsäure zugesetzt, dass die Natronlauge im Gemisch neutralisiert wird und das ablaufende, wässrige Gemisch einen pH Wert von 4 - 5 aufweist.

Das Aceton wird wieder in den Prozess zurückgeführt.

Nach Abtrennen der wässrigen Phase wird bei ca. 100°C und ca. 50 mbar getrocknet und in einer Trennwandkolonne mit 2 Seitenabzügen destillativ gereinigt. Am unteren Seitehabzug werden ca. 400 kg/h Pseudojonon mit einer Reinheit von 98% erhalten (Flächen-% GLC, Summe aller lsomere!). Am oberen Seitenabzug fallen ca. 26 kg/h Citral an (Summe aller Isomere), die kontinuierlich in den Prozess zurückgeführt werden.

### Beispiel 2:

### Methylpseudojonone

110kg/h Citral (0,72 kmol/h), ca. 20 kg/h Rückcitral (0,13 kmol/h), 1.800 kg/h wässriges 82%iges 2-Butanon und ca. 20 kg/h 5%ige wässrige NaOH werden vermischt. Es entsteht eine homogene Lösung, die vorsorglich über einen Phasenabscheider geleitet wird. Ist der Wasseranteil oder auch der NaOH-Anteil höher als angegeben, kann sich nach dem Mischprozess eine wässrige Phase abtrennen, die abgeschieden werden muss. Das Gemisch wird auf 136°C geheizt und durch einen 160 l Rohrreaktor gepumpt. Die Reaktionstemperatur erwärmt das Gemisch weiter auf ca. 138°C. In 4 Minuten Verweilzeit wird bezogen auf Citral ein Umsatz von ca. 82% erzielt.

Die Reaktionsmischung aus dem Rohrreaktor wird auf Normaldruck entspannt. Hierbei destillieren ca. 1000 l/h 2-Butanon ab, wobei sich die Produktlösung auf ca. 75°C abkühlt. Anschließend wird im Gegenstrom mit ca. 550 kg/h Dampf von restlichem 2-Butanon befreit. Dem Dampf werden so viel Essigsäure zugesetzt, dass die Natronlauge im Gemisch neutralisiert wird und das ablaufende, wässrige Gemisch einen pH Wert von 4 - 5 aufweist.

Das 2-Butanon wird wieder in den Prozess zurückgeführt.

Nach Abtrennen der wässrigen Phase wird bei ca. 100°C und ca. 50 mbar getrocknet und in einer Trennwandkolonne mit 2 Seitenabzügen destillativ gereinigt. Am unteren Seitenabzug werden ca. 100 kg/h Methylpseudojonone mit einer Reinheit von 98% erhalten (Flächen-% GLC, Summe aller Isomere!). Am oberen Seitenabzug fallen ca. 20 kg/h Citral an (Summe aller lsomere), die kontinuierlich in den Prozess zurückgeführt werden.

Es wird ein Isomerenverhältnis von etwa n : iso = 5 : 1 erhalten.

### Beispiel 3

### Methylpseudojonone

100 kg/h Citral (0,66 kmol/h), ca. 25 kg/h rückgeführtes Citral (0,16 kmol/h), 2200 I/h wässriges 88%iges 2-Butanon und ca. 120 kg/h 40%ige wässrige NaOH werden vermischt. Es entsteht eine zweiphasige Lösung, die über einen Phasenabscheider geleitet wird. Ca. 120 kg/h wässrige NaOH werden abgetrennt und ca. 2100 kg/h organische Phase auf 120°C geheizt und durch einen 160 l Rohrreaktor gepumpt. Die Reaktionstemperatur erwärmt das Gemisch weiter auf ca. 132°C. Bei einer Verweilzeit von 4 Minuten wird bezogen auf Citral ein Umsatz von ca. 75% erzielt.

Die Reaktionsmischung aus dem Rohrreaktor wird auf Normaldruck entspannt. Hierbei destillieren ca. 1000 l/h 2-Butanon ab, wobei sich die Produktlösung auf ca. 75°C abkühlt. Anschließend wird im Gegenstrom mit ca. 550 kg/h Dampf von restlichem 2-Butanon befreit. Dem Dampf wird so viel Essigsäure zugesetzt, dass die Natronlauge im Gemisch neutralisiert wird und das ablaufende, wässrige Gemisch einen pH Wert von 4 - 5 aufweist.

Überschüssiges 2-Butanon wird wieder in den Prozess zurückgeführt.

Nach Abtrennen der wässrigen Phase wird bei ca. 100°C und ca. 50 mbar getrocknet und in einer Trennwandkolonne mit 2 Seitenabzügen destillativ gereinigt. Am unteren Seitenabzug werden ca. 100 kg/h Methylpseudojonone mit einer Reinheit von 98% erhalten (Flächen-% GLC, Summe aller Isomere). Am oberen Seitenabzug fallen ca. 25 kg/h Citral an (Summe aller Isomere), die kontinuierlich in den Prozess zurückgeführt werden.

Es wird ein lsomerenverhältnis von etwa n : iso = 8 :1 erhalten.

### Beispiel 4

### Methyljonone

Ca. 140 l/h Methylpseudojonone, 400 l/h Hexan (auf -8°C vorgekühlt!) und 200 l/h 89%ige Schwefelsäure werden hintereinander in einer Reaktionspumpe innig vermischt. Das Gemisch erwärmt sich spontan auf ca. 29°C, wird auf etwa 26°C gekühlt und bei dieser Temperatur ca. 2 min in einer Verweilzeitstrecke umgepumpt, bevor es in einer weiteren Reaktionspumpe mit ca. 600 l/h Wasser verdünnt wird. Das Gemisch erwärmt sich durch die Wasserzugabe auf ca. 47°C und wird über einen nachfolgenden Kühler auf < 45°C gehalten. Nach Abtrennen der stark schwefelsauren Wasserphase und einer weiteren Wasserwäsche wird im Gegenstrom mit Dampf das Hexan entfernt. Das Hexan wird wieder in die Reaktion zurückgeführt.

Nach Abtrennen der wässrigen Phase,wird bei ca. 50 mbar und 100°C getrocknet und in einer Trennwandkolonne mit 2 Seitenabzügen gereinigt. Im Hauptlauf (Seitenabzug 1) werden ca. 120 l/h Methyljonone mit einem Gehalt an β-n-Methylpseudojonon zwischen 80 und 90% erhalten.

In nachfolgender Tabelle ist eine der typischen Zusammensetzungen aufgelistet¹⁾:
¹⁾ E- und Z-lsomere werden gemeinsam ausgewiesen, wobei die E-lsomeren dominieren.

| | | | |
|---|---|---|---|
| α-iso-Methyljonon | 8,8 % ± 1 | Summe Iso-Methyljonone | 10,7 % ± 1,6 |
| β-iso-Methyljonon | 0,8 % ± 0,1 | | |
| γ-iso-Methyljonon | 1,1 % ± 0,5 | | |

| | | | |
|---|---|---|---|
| α-n-Methyljonon | 0,25 % ± 0,1 | Summe n-Methyljonone | 85,5 % % ± 1,8 |
| β-n-Methyljonon | 85,0 % ± 1,5 | | |
| γ-n-Methyljonon | 0,3 % ± 0,2 | | |

## Patentansprüche

1. Kontinuierliches Verfahren zur Herstellung von Pseudojononen der allgemeinen Formeln I bzw. I' sowie ihrer Isomeren 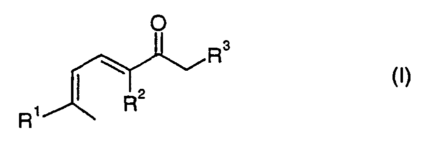 bzw. 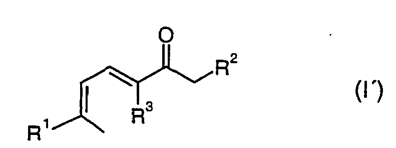 wobei R¹
CH₃ oder 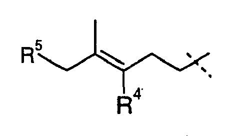 bedeutet,
R², R³ Wasserstoff, CH₃ oder C₂H₅ bedeuten,
R⁴, R⁵ Wasserstoff oder CH₃ bedeuten,
durch Umsetzen eines Aldehyds der Formel (II), 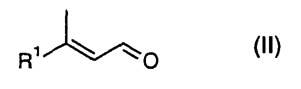 mit einem Überschuss eines Ketons der allgemeinen Formel (III) 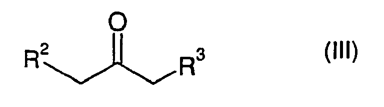 wobei R¹, R² und R³ die oben angegebenen Bedeutungen besitzen, in Gegenwart von Wasser und Alkalihydroxid bei erhöhter Temperatur in homogener Lösung, **dadurch gekennzeichnet, dass**
a) die Durchmischung der homogenen Lösung von Aldehyd, Keton und wässriger Alkalilauge bei einer Temperatur von 10 bis 120°C stattfindet, dann
b) das nicht im Reaktionsgemisch gelöste Wasser und Alkalihydroxid abgetrennt werden,
c) anschließend das homogene Reaktionsgemisch unter Vermeidung von Rückvermischung bei einer Temperatur, die 10 bis 120°C oberhalb des Siedepunktes der am niedrigsten siedenden Komponente und einem Dampfdruck p von 10⁶ bis 10⁷ Pa durch einen Reaktor geleitet wird, der eine Verweilzeit von 2 bis 300 Minuten ermöglicht,
d) das Reaktionsgemisch unter Entspannung abgekühlt wird,
e) das Keton mit Dampf im Gegenstrom aus dem Reaktionsgemisch entfernt wird und
f) das Rohprodukt getrocknet und über eine Rektifikationskolonne von überschüssigem Aldehyd und Nebenkomponenten befreit wird.

2. Verfahren nach Anspruch 1, **dadurch gekennzeichnet, dass** die Ketonkomponente der allgemeinen Formel (II) im 5- bis 50-fachen molaren Überschuss zugegeben wird, wobei der nicht umgesetzte Anteil nach der Reaktionsstrecke bei einem Druck von 10⁷ bis 5·10⁸ mPa_{abs}. abgetrennt und dem Frischketon zur Synthese wieder zugeführt wird.

3. Verfahren nach Anspruch 1 und 2, **dadurch gekennzeichnet, dass** die Reaktionstemperatur bei gegebener Verweilzeit so gewählt wird, dass der Umsatz der Aldehydkomponente 60 bis 98% beträgt, und der nicht umgesetzte Aldehyd abgetrennt und in die Umsetzung rückgeführt wird.

4. Verfahren nach den Ansprüchen 1 bis 3, **dadurch gekennzeichnet, dass** der Wassergehalt des zur Reaktion eingesetzten Ketons der Formel (lll) zwischen 1 und 15 Gew.-% liegt.

5. Verfahren nach den Ansprüchen 1 bis 4, **dadurch gekennzeichnet, dass** die Konzentration des zur Reaktion eingesetzten Alkalihydroxids zwischen 0,005 und 50 Gew.%, vorzugsweise 5 -10 Gew.% liegt.

6. Verfahren nach den Ansprüchen 1 bis 5 zur Herstellung von Pseudoiononen der Formel I sowie ihrer Isomeren, wobei R² oder R³ Methyl bedeuten, **dadurch gekennzeichnet, dass** die Konzentration des zur Reaktion eingesetzten Alkalihydroxids 10 bis 50 Gew.-%, vorzugsweise 35 bis 45 Gew.-% beträgt.

7. Verfahren nach den Ansprüchen 1 bis 6, **dadurch gekennzeichnet, dass** das eingesetzte Keton der Formel (III) im wesentlichen aus nach der Reaktion abgetrenntem, überschüssigem Keton der Formel (III) mit einem Wassergehalt von 1-15 Gew.-% besteht, dem sowohl wasserfreies als auch wasserhaltiges Keton der Formel (III) mit einem Wassergehalt von 1-15 Gew.-% ergänzt werden darf.

8. Verfahren nach den Ansprüchen 1 bis 7, **dadurch gekennzeichnet, dass** bei Umsatz mit Ketonen der allgemeinen Formel (III) mit R² ≠ H und R³ = H ein Produktgemisch erhalten wird, welches 70 bis 95% n-Alkylpseudojonone und 5 bis 30% iso-Alkylpseudojonone enthält

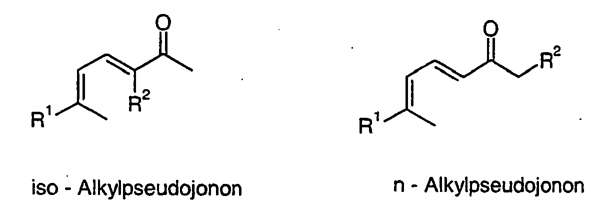

9. Verfahren nach den Ansprüchen 1-8, **dadurch gekennzeichnet, dass** die nach Anspruch 1 bis 8 erhaltenen Pseudojonone zu Jononen der allgemeinen Formeln (IV) bis (VI) 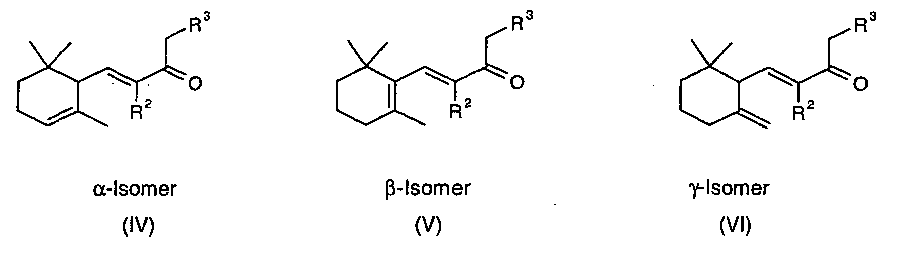 umgesetzt werden, in der Form, dass das Verhältnis der n-Form (R² = H, R³ = Alkyl) zur iso-Form (R² = Alkyl, R³ = H) gemäß Anspruch 8 erhalten bleibt.

10. Verfahren nach Anspruch 9, **dadurch gekennzeichnet, dass** die nach Anspruch 1 bis 8 erhaltenen Pseudojonone mit hochprozentiger Schwefelsäure in Gegenwart eines unter den Reaktionsbedingungen inerten Verdünnungsmittels zu Jononen umgesetzt werden, wobei die Reaktionstemperatur 0-20°C und die Verweilzeit zwischen Zyklisierung und Hydrolyse 10 bis 300 Sekunden, vorzugsweise 120 Sekunden beträgt.

## Claims

1. A continuous process for preparing pseudoionones of the general formulae I or I' and isomers thereof 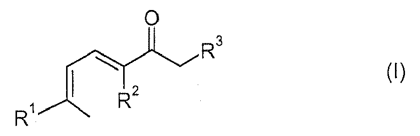 or 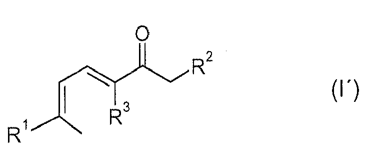 where R¹ is
CH₃ or 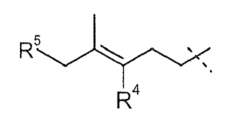 R², R³ are each hydrogen, CH₃ or C₂H₅,
R⁴, R⁵ are each hydrogen or CH₃,
by reacting an aldehyde of the formula (II) 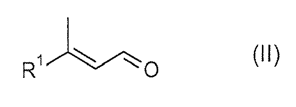 with an excess of a ketone of the general formula (III) 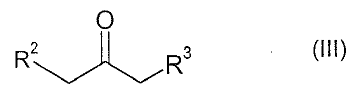 where R¹, R² and R³ are each as defined above, in the presence of water and alkali metal hydroxide at elevated temperature in homogeneous solution, which comprises
a) mixing the homogeneous solution of aldehyde, ketone and aqueous alkali metal hydroxide at a temperature of from 10 to 120°C, then
b) removing the water and alkali metal hydroxide which have not dissolved in the reaction mixture,
c) subsequently passing the homogeneous reaction mixture, avoiding backmixing, at a temperature which is from 10 to 120°C above the boiling point of the lowest-boiling component and a vapor pressure p of from 10⁶ to 10⁷ Pa through a reactor which enables a residence time of from 2 to 300 minutes,
d) cooling the reaction mixture under decompression,
e) removing the ketone from the reaction mixture with steam in countercurrent and
f) drying the crude product and freeing it of excess aldehyde and secondary components using a rectification column.

2. The process according to claim 1, wherein the ketone component of the general formula (II) is added in a from 5- to 50-fold molar excess, and the unconverted proportion, downstream of the reaction zone, is removed at a pressure of from 10⁷ to 5·10⁸ mPa_{abs}. and added again to the fresh ketone for the synthesis.

3. The process according to claim 1 or 2, wherein the reaction temperature at a given residence time is selected in such a way that the conversion of the aldehyde component is from 60 to 98%, and the unconverted aldehyde is removed and recycled into the reaction.

4. The process according to claims 1 to 3, wherein the water content of the ketone, used for the reaction, of the formula (III) is between 1 and 15% by weight.

5. The process according to claims 1 to 4, wherein the concentration of the alkali metal hydroxide used for the reaction is between 0.005 and 50% by weight, preferably 5 - 10% by weight.

6. The process according to claims 1 to 5 for preparing pseudoionones of the formula I and isomers thereof where R² or R³ is methyl, wherein the concentration of the alkali metal hydroxide used for the reaction is from 10 to 50% by weight, preferably from 35 to 45% by weight.

7. The process according to claims 1 to 6, wherein the ketone of the formula (III) used consists substantially of excess ketone of the formula (III) which has been removed from the reaction and has a water content of 1 - 15% by weight, which may be supplemented with either anhydrous or aqueous ketone of the formula (III) having a water content of 1 15% by weight.

8. The process according to claims 1 to 7, wherein, in the case of reaction with ketones of the general formula (III) where R² ≠H and R³ = H, a product mixture is obtained which contains from 70 to 95% n-alkylpseudoionones and from 5 to 30% isoalkylpseudoionones

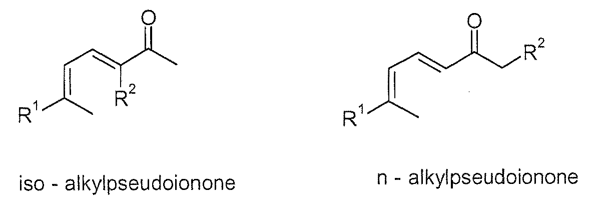

9. The process according to claims 1 to 8, wherein the pseudoionones obtained according to claims 1 to 8 are converted to ionones of the general formulae (lV) to (Vl) 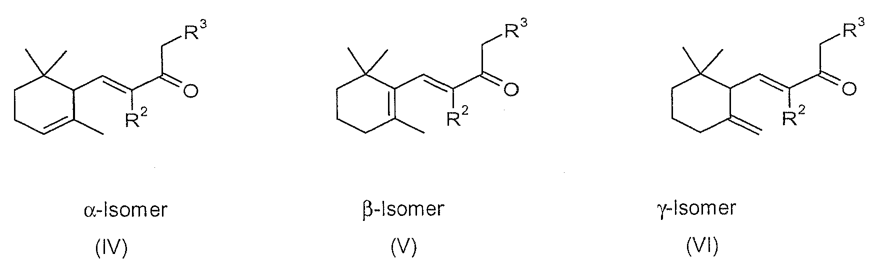 in the form such that the ratio of the n-form (R² = H, R³ = alkyl) to the iso-form (R² = alkyl, R³ = H) according to claim 8 is maintained.

10. The process according to claim 9, wherein the pseudoionones obtained according to claims 1 to 8 are reacted with highly concentrated sulfuric acid in the presence of a diluent which is inert under the reaction conditions to give ionones, the reaction temperature being 0-20°C and the residence time between cyclization and hydrolysis being from 10 to 300 seconds, preferably 120 seconds.

## Revendications

1. Procédé continu de préparation de pseudoionones de la formule générale I ou respectivement I' ainsi que de leurs isomères : 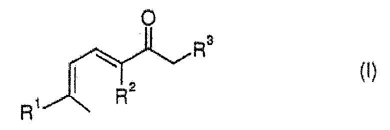 ou respectivement 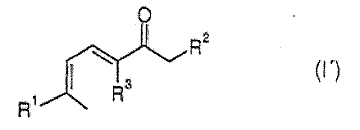 dans lesquelles R¹ représente
CH₃ ou 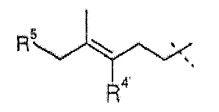
R², R³ représentent de l'hydrogène, CH₃ ou C₂H₅,
R⁴, R⁵ représentent de l'hydrogène ou CH₃,
par réaction d'un aldéhyde de la formule (II) : 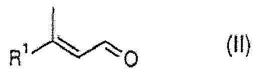 avec un excès d'une cétone de la formule générale (III) : 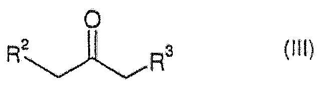 où R¹, R² et R³ possèdent les significations données ci-dessus, en présence d'eau et d'hydroxyde de métal alcalin à une température élevée, en solution homogène, **caractérisé en ce que**
a) le mélange intime de la solution homogène d'aldéhyde, de cétone et de lessive aqueuse d'alcali a lieu à une température de 10 à 120°C, puis
b) l'eau et l'hydroxyde de métal alcalin non dissous dans le mélange réactionnel sont isolés,
c) ensuite le mélange réactionnel homogène est, en évitant un remélangeage, conduit à une température de 10 à 120°C, au-dessus du point d'ébullition du composant à point d'ébullition le plus bas, et à une tension de vapeur p de 10⁶ à 10⁷ Pa, au travers d'un réacteur qui permet un temps de séjour de 2 à 300 minutes,
d) le mélange réactionnel est refroidi par détente,
e) la cétone est éliminée du mélange réactionnel avec de la vapeur d'eau en contre-courant, et
f) le produit brut est séché et libéré de l'aldéhyde excédentaire et de composants secondaires en le passant par une colonne de rectification.

2. Procédé suivant la revendication 1, **caractérisé en ce que** le composant cétone de la formule générale (II) est ajouté en un excès molaire de 5 à 50 fois, la fraction n'ayant pas réagi après la zone réactionnelle étant isolée à une pression de 10⁷ à 5·10⁸ mPa_{abs}. et étant ramenée à la cétone fraîche pour la synthèse.

3. Procédé suivant la revendication 1 ou 2, **caractérisé en ce que** la température réactionnelle pour un temps de séjour donné est choisie de façon que la conversion du composant aldéhyde soit de 60 à 98 % et l'aldéhyde n'ayant pas réagi est isolé et reconduit dans la réaction.

4. Procédé suivant les revendications 1 à 3, **caractérisé en ce que** la teneur en eau de la cétone de la formule (III) mise en oeuvre pour la réaction est comprise entre 1 et 15 % en poids.

5. Procédé suivant les revendications 1 à 4, **caractérisé en ce que** la concentration de l'hydroxyde de métal alcalin mis en oeuvre pour la réaction est comprise entre 0,005 et 50 % en poids, de préférence 5 et 10 % en poids.

6. Procédé suivant les revendications 1 à 5, pour la préparation de pseudoionones de la formule I ainsi que de leurs isomères, où R² ou R³ représentent du méthyle, **caractérisé en ce que** la concentration de l'hydroxyde de métal alcalin mis en oeuvre pour la réaction est comprise entre 10 et 50 % en poids, de préférence 35 et 45 % en poids.

7. Procédé suivant les revendications 1 à 6, **caractérisé en ce que** la cétone de la formule (III) mise en oeuvre est essentiellement constituée de cétone de la formule (III) excédentaire, isolée après la réaction, qui présente une teneur en eau de 1-15 % en poids, de la cétone de la formule (III) anhydre aussi bien que contenant de l'eau pouvant être complétée à une teneur en eau de 1-15 % en poids.

8. Procédé suivant les revendications 1 à 7, **caractérisé en ce que**, lors de la réaction avec des cétones de la formule générale (III) où R² ≠ H et R³ = H, un mélange produit est obtenu qui contient 70 à 95 % de n-alkylpseudoionones et 5 à 30 % d'iso-alkylpseudoionones :

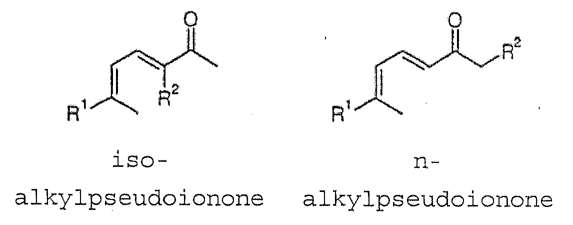

9. Procédé suivant les revendications 1 à 8, **caractérisé en ce que** les pseudoionones obtenues selon les revendications 1 à 8 sont converties en ionones des formules générales (IV) à (VI) : 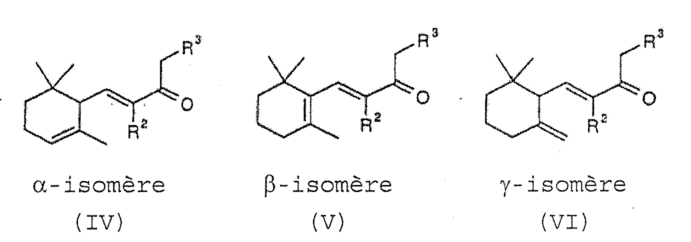 sous une forme telle que le rapport entre la forme n (R² = H, R³ = alkyle) et la forme iso (R² = alkyle, R³ = H) selon la revendication 8 reste maintenu.

10. Procédé suivant la revendication 9, **caractérisé en ce que** les pseudoionones obtenues selon les revendications 1 à 8 sont mises à réagir avec de l'acide sulfurique à haut pourcentage, en présence d'un diluant inerte dans les conditions réactionnelles, pour former des ionones, la température réactionnelle étant de 0-20°C et le temps de séjour entre la cyclisation et l'hydrolyse de 10 à 300 secondes, de préférence de 120 secondes.
